# FASCICULE DE BREVET EUROPEEN

(11) **EP 0 689 808 B1**
(45) Date de publication et mention de la délivrance du brevet: **15.09.1999**
(21) Numéro de dépôt: 95420142.2
(22) Date de dépôt: 02.06.1995
(51) Int. Cl.: A61F 2/38

(54) **Ensemble prothétique de l'articulation du genou**
Prothesensatz für das Kniegelenk
Knee joint prosthetic set

(30) Priorité: 03.06.1994 FR 9407088
(43) Date de publication de la demande: 03.01.1996
(73) Titulaire: PROVIS S.A., 69008 Lyon (FR)
(72) Inventeur: Boudarel,Patrick, F-69390 Charly (FR); Boudarel, René, F-97150 Saint Martin (FR); Dumontier Philippe, F-03000 Bressoles (FR); Nael Jean Francois, F-03000 Bressoles (FR); Danan Jean Paul, F-91570 Bievres (FR); Bottau Alain, F-42600 Chalain le Comtal (FR); Mesgish Alain, F-42570 Saint Heand (FR)
(74) Mandataire: Thivillier, Patrick

(56) Documents cités:
- EP-A- 0 562 782
- FR-A- 2 672 798
- US-A- 4 892 547
- US-A- 4 936 853
- US-A- 5 147 405

## Description

L'invention concerne le secteur technique des prothèses du genou, plus particulièrement des prothèses tricompartimentales dites à glissement.

De manière parfaitement connue, ce type de prothèses comprend un implant fémoral et un implant tibial. L'implant tibial présente un plateau, généralement en polyéthylène, fixé sur une embase tibiale, généralement métallique. Le plateau tibial reçoit, en appui et à glissement, les patins condyliens de l'implant fémoral.

De même, en fonction du cas pathologique à traiter, l'implant tibial peut présenter, du côté de son bord postérieur, une échancrure pour le passage des ligaments croisés postérieurs. Le plateau tibial peut également présenter différentes configurations, c'est le cas notamment, d'une prothèse postéro-stabilisée où le plateau présente, dans sa partie médiane, un bossage apte à coopérer avec les agencements complémentaires de l'implant fémoral, en vue de son guidage au moment de la flexion.

Généralement, il est nécessaire d'avoir auant d'embases tibiales qu'il y a de variétés possibles de plateaux. Or, il serait parfois intéressant de disposer d'une embase tibiale commune, sur laquelle pourrait être montés des types différents de plateaux, correspondant très exactement au cas pathologique à traiter. Cependant, la fixation du plateau sur l'embase, selon l'état de la technique connue, ne permet pas, d'une manière aisée, une telle interchangeabilité.

En effet, dans la plupart des cas, l'accouplement entre le plateau et l'embase s'effectue par emboitement. Dans ce but, l'embase présente un rebord périphérique présentant des agencements de retenue, aptes à coopérer avec des agencements complémentaires du plateau. Un tel système d'emboitement, compte-tenu de l'effort nécessaire qu'il convient d'appliquer, ne peut s'effectuer qu'en dehors du champ opératoire. Parfois, ce mode d'accouplement est combiné avec une fixation par vis.

On connait également par l'enseignement du brevet US 4936853, une prothèse pour l'articulation du genou, où l'engagement du plateau tibial sur l'embase correspondante, s'effectue par la combinaison d'un système de rainures et de nervures complémentaires et d'un ergot que présente en débordement, la face de dessous du plateau et coopérant avec une encoche de l'embase. De telles dispositions rendent difficile l'accouplement du plateau par rapport à l'embase. Il en est de même si l'on veut procéder au démontage du plateau.

Le problème que se propose de résoudre l'invention, est de réaliser un ensemble prothétique, de l'articulation du genou, entièrement modulable, afin d'avoir un nombre d'éléments réduits, avec la capacité de les combiner entre eux, pour répondre au maximum de cas pathologiques à traiter.

Notamment, un problème que se propose de résoudre l'invention, est de pouvoir équiper une embase tibiale commune, de différents types de plateaux, avec des agencements d'accouplement appropriés, compte-tenu de cette possibilité d'interchangeabilité, tout en ayant pour but d'avoir une fixation parfaitement sûre du plateau par rapport à l'embase, c'est-à-dire avoir une fixation postérieure, antérieure et latérale, tout en ayan tpour objectif d'assurer le guidage du plateau au moment de sa mise en place sur l'embase tibiale.

Pour résoudre un tel problème, il a été conçu et mis au point un ensemble prothétique du type de ceux comprenant un implant fémoral et un implant tibial sous forme d'un plateau en polyéthylène, monté en combinaison avec une embase métallique. Selon l'invention, il est revendiqué que l'embase métallique présente un moyen d'accouplement et de guidage constitué par une nervure de section transversale profilée en T, formée à partir du bord antérieur de l'embase, sur une longueur limitée et apte à coopérer avec une rainure de forme complémentaire, établie dans l'axe médian de la face de dessous du plateau, permettant ainsi l'engagement dudit plateau par simple coulissement, à partir du bord antérieur de l'embase jusqu'à une position de butée, l'ensemble du plateau et de l'embase étant percés coaxialement pour l'engagement d'une vis de blocage.

Pour résoudre le problème posé d'assurer la fixation du plateau tibial par rappport à l'embase, la face de dessous de l'embase présente en débordement et en alignement coaxial avec les trous pour le passage de la vis de blocage, une portée de centrage creuse apte à recevoir une quille de stabilité quelconque conformée pour le vissage de ladite vis.

La position de butée est obtenue par un rebord équerré formé à partir du bord postérieur de l'embase et coopérant avec une échancrure complémentaire, formée au niveau de la face de dessous du plateau.

Il apparait donc que la face de dessus de l'embase tibiale est entièrement lisse, à l'exception d'une partie de son bord postérieur, offrant ainsi une grande surface d'appui pour le plateau.

Pour résoudre le problème posé d'assurer la fixation de la quille par rapport à l'embase tibiale, la portée de centrage pour le montage de la quille de stabilité, a une forme tronconique, en étant apte à être engagée dans un chambrage que présente ladite quille, le fond du chambrage étant taraudé pour recevoir la vis de blocage.

Dans le cas d'une quille à ailettes, l'embase et la quille présentent des moyens complémentaires d'indexation angulaire.

Pour résoudre le problème posé d'éviter toute désolidarisation du plateau par rapport à l'embase, la vis de blocage présente une tête délimitant une portée tronconique, conformée pour être engagée dans un alésage complémentaire du plateau, afin d'être mise en force dans ledit plateau et participer à son expansion.

Avantageusement, la fixation de l'ensemble de l'implant tibial, peut être complétée par des chevilles expansives ou des vis d'ostéosynthèse.

Dans ce but, l'embase tibiale qui est entièrement symétrique, présente des trous pour l'engagement des chevilles expansives, ou des vis d'ostéosynthèse.

Toujours en ayant pour objectif d'utiliser des pièces standards et d'avoir une embase commune, les trous reçoivent des rondelles de formes tronconiques, pour être emmanchées dans lesdits trous, lesdites rondelles formant chambrage interne pour le logement des têtes de vis d'ostéosynthèse.

Avantageusement, l'implant tibial tel que défini, reçoit en appui et à glissement, un implant fémoral qui présente des patins condyliens présentant une portée évolutive, d'épaisseur distale postérieure identique, lesdits patins étant raccordés par une partie faisant office de trochlée, et qui délimite une surface d'appui concave, convenablement orientée pour recevoir une rotule, afin d'encaisser un maximum d'effort aux moyennes flexions.

L'invention est exposée, ci-après plus en détail à l'aide des dessins annexés, dans lesquels :

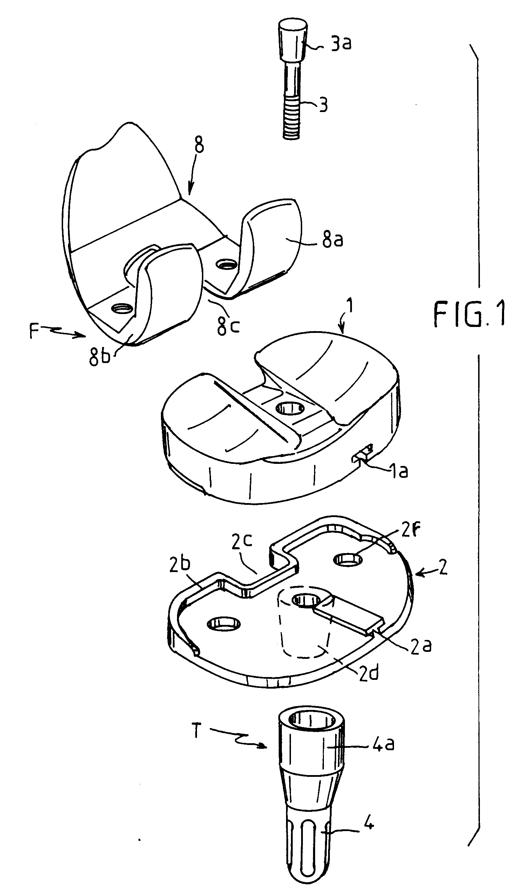

La figure 1 est une vue en perspective avant assemblage, des principaux éléments de l'ensemble prothétique selon l'invention.
La figure 2 est une vue en plan de l'embase tibiale.
La figure 3 est une vue correspondant à la figure 2, montrant le montage d'un plateau tibial standard.
La figure 4 est une vue correspondant à la figure 3, dans le cas d'un plateau tibial stabilisé.
La figure 5 est une vue en coupe considérée selon la ligne 5.5 de la figure 3.
La figure 6 est une vue en coupe considérée selon la ligne 6.6 de la figure 3.
La figure 7 est une vue en coupe considérée selon la ligne 7.7 de la figure 4.
La figure 8 est une vue en coupe considérée selon la ligne 8.8 de la figure 4.
Les figures 9, 10, 11, 12, 13 et 14 montrent différentes formes de réalisations, de quilles de stabilité et d'ancrage
La figure 15 est une vue en plan correspondant à la figure 14.
La figure 16 est une vue de face de l'implant fémoral.
La figure 17 est une vue de côté, correspondant à la figure 16.
La figure 18 est une vue en plan correspondant à la figure 17.
La figure 19 est une vue montrant l'implant fémoral équipé d'un module de stabilistion, dans le cas d'une prothèse postéro-stabilisée.
La figure 20 est une vue en plan correspondant à la figure 19.

L'ensemble prothétique comprend, selon l'invention, d'une manière connue, un implant fémoral (F) et un implant tibial (T), conformés pour constituer une prothèse dite tricompartimentale. L'implant tibial comprend un plateau (1), en polyéthylène, conformé pour être monté dans une embase (2), généralement métallique.

Selon une première caractéristique, l'embase (2) est conformée pour recevoir, à libre coulissement, à la façon d'un tiroir, le plateau (1). La surface d'appui de l'embase (2) recevant le plateau (1), est entièrement lisse et présente, à partir de son bord antérieur, une nervure (2a). Cette nervure a une section transversale profilée en T et est établie selon l'axe médian de l'embase, selon une longueur limitée, s'étendant très sensiblement du bord antérieur, au centre de ladite embase. La nervure (2a) coopère avec une rainure de forme complémentaire (1a), établie dans l'axe médian de la face de dessous du plateau (1).

Après engagement du plateau (1) sur l'embase (2), par coulissement de la rainure (1a) sur la nervure (2a), ledit plateau (1) vient en position de butée, avec des agencements du bord postérieur de l'embase (2). Ces agencements de butée sont constitués par un rebord équerré (2b) établi à partir du bord postérieur de l'embase, en s'étendant partiellement sur les côtés latéraux de cette dernière. Ce rebord équerré (2b) coopère avec une échancrure complémentaire (1b), formée au niveau de la face de dessous du plateau (1).

A noter que le plateau (1) et l'embase (2) peuvent présenter une échancrure médiane (1c) (2c), pour le passage des ligaments croisés postérieurs.

Après accouplement du plateau (1) sur l'embase (2), dans les conditions indiquées ci-dessus, ces derniers sont rendus solidaires au moyen d'une vis de blocage (3), apte à être vissée dans une quille centrale de stabilité et d'ancrage (4). Dans ce but, le plateau (1) et l'embase (2) sont percés coaxialement en alignement avec une portée de centrage creuse (2d) apte à recevoir la quille (4), qui peut être de conception quelconque, comme il sera indiqué ci-après.

La portée (2d), établie coaxialement en débordement de la face de dessous de l'embase (2), a une forme tronconique apte à être engagée dans un chambrage (4a) que présente la quille (4). Le fond du chambrage (4a) est taraudé pour recevoir la vis de blocage (3).

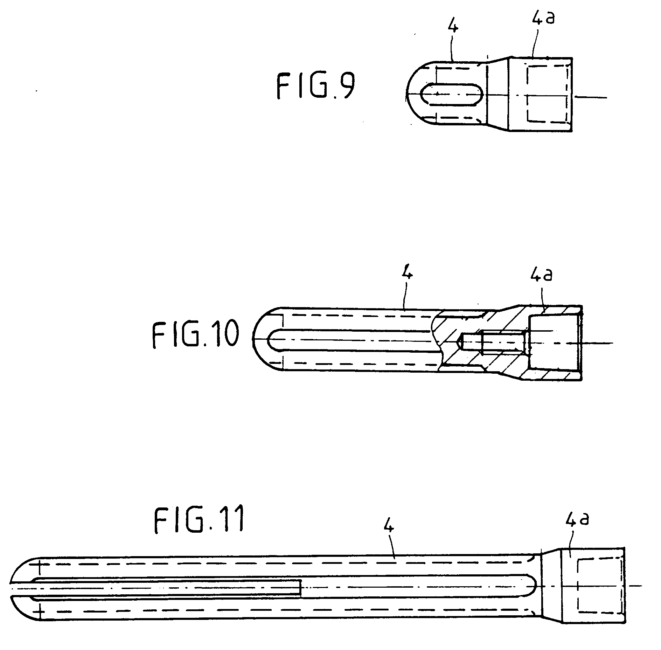

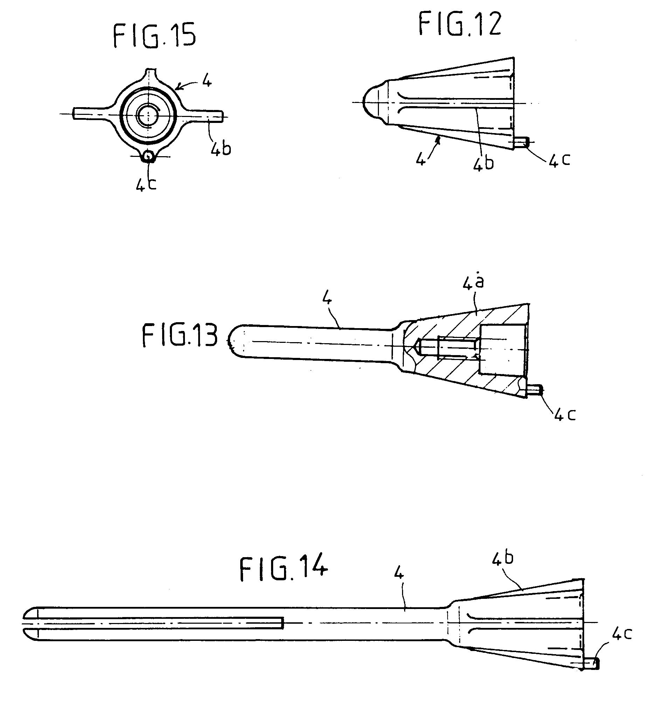

Comme le montrent les figures 10, 11, 12, 13, 14,15, la quille (4) peut présenter différentes formes de réalisation et être exécutée dans différentes longueurs, en fonction du cas pathologique à traiter. Par exemple, la quille (4) peut présenter des ailettes (4b). Dans ce cas, l'embase tibiale (2) et la quille (4) présentent des moyens complémentaires d'indexation angulaires, sous forme, par exemple, d'un pion (4c) coopérant avec un trou (2e) de l'embase. Pour des quilles de grande longueur, ces dernières peuvent être fendues (figures 12 et 15).

La vis de blocage (3) présente une tête (3a) délimitant une portée tronconique, conformée pour être engagée dans un alésage complémentaire de l'embase (2), afin d'être mise en force dans le plateau (1) et participer ainsi à son expansion. Il apparait donc qu'après accouplement du plateau (1) sur l'embase (2), suite à l'engagement de la rainure (1a) sur la nervure (2a), et blocage dudit plateau sur ladite embase, au moyen de la vis (3), vissée dans la quille (4) dans les conditions indiquées, on obtient une parfaite stabilité du plateau, dans les plans antérieur, postérieur et latéral.

L'embase (2) présente des trous (2f) pour l'engagement de chevilles expansives ou de vis d'ostéosynthèse (5). Ces trous peuvent être au nombre de deux, en étant disposés symétriquement par rapport aux agencements de fixation de la quille (4). Les chevilles expansives ne sont pas illustrées et sont de tous types connus et appropriés.

En ce qui concerne les vis d'ostéosynthèse (5), qui sont d'un diamètre normalisé, il est nécessaire de prévoir des rondelles (7) présentant un chambrage interne, pour le logement des têtes desdites vis. Ces rondelles (7) sont engagées dans les trous (2f). Il apparait donc que les mêmes trous (2f) peuvent être utilisés indiféremment pour des chevilles d'expansion et des vis d'ostéosynthèse, malgré leur différence de diamètre.

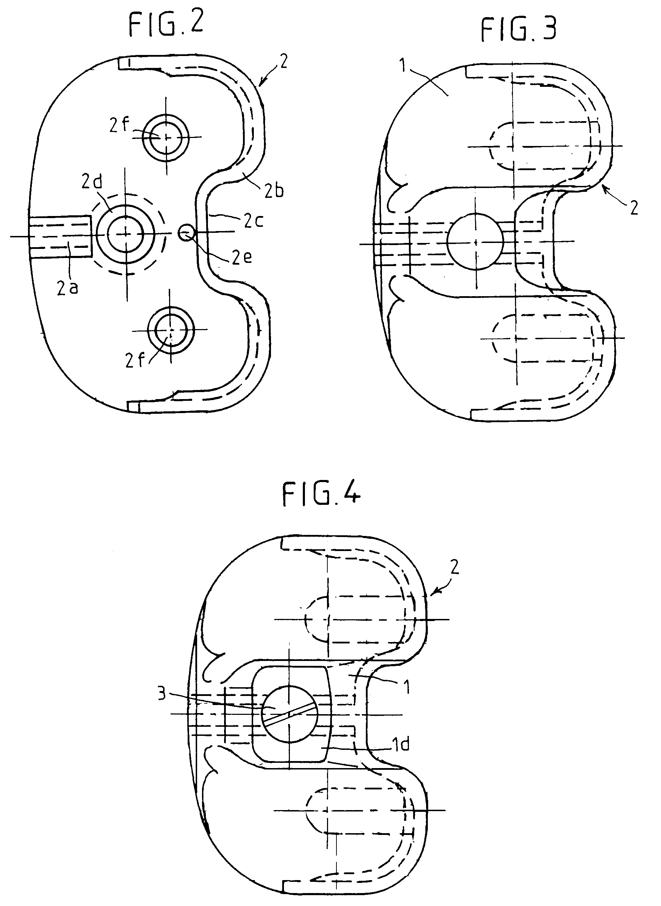

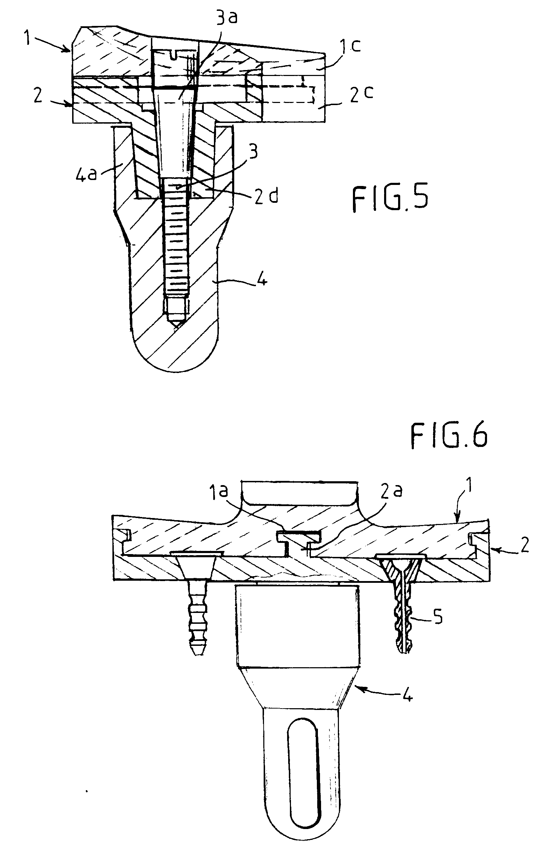

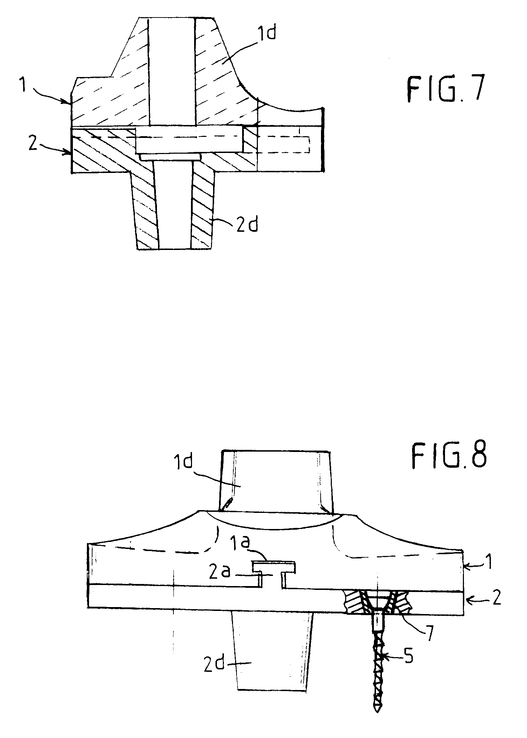

De même, le plateau en polyéthylène (1) peut être du type standard (figures 3, 4, 5) ou présenter un bossage central profilé (1d), dans le cas d'une prothèse postéro-stabilisée (figures 6, 7, 8).

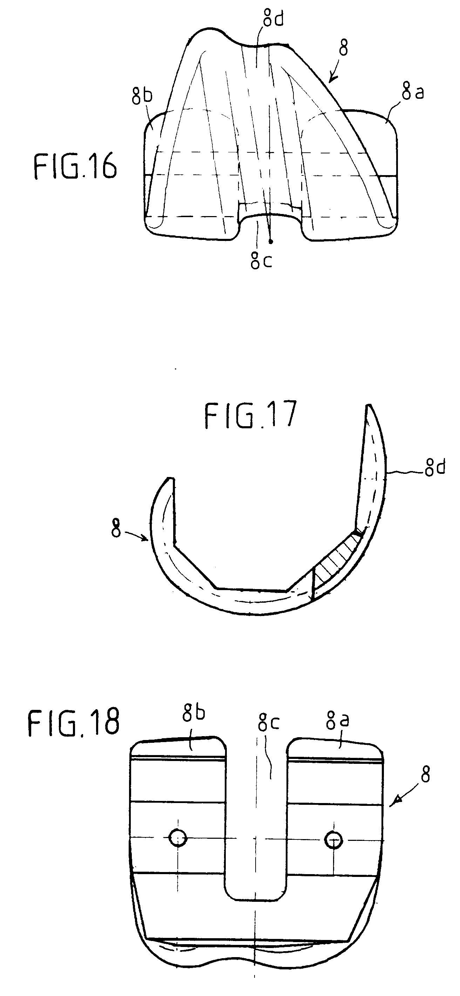

En ce qui concerne l'implant fémoral (F) (figures 16, 17, 18), ce dernier constitue un corps monobloc (8) présentant des patins condyliens (8a) (8b) séparés par une échancrure intercondylienne (8c). Ces patins présentent une portée évolutive d'épaisseur distale et postérieure identique. Les patins condyliens (8a) et (8b) sont raccordés par une partie (8d) faisant office de trochlée, présentant une surface d'appui concave, convenablement orientée pour recevoir un bouton rotulien.

Ces dispositions permettent à l'élément fémoral d'encaisser un maximum d'efforts aux moyennes flexions. Le bouton rotulien rerouve une certaine mobilité vers l'hyper extension.

Le bouton rotulien n'est pas décrit et illustré, car il peut présenter différentes formes de réalisation parfaitement connues pour un homme du métier.

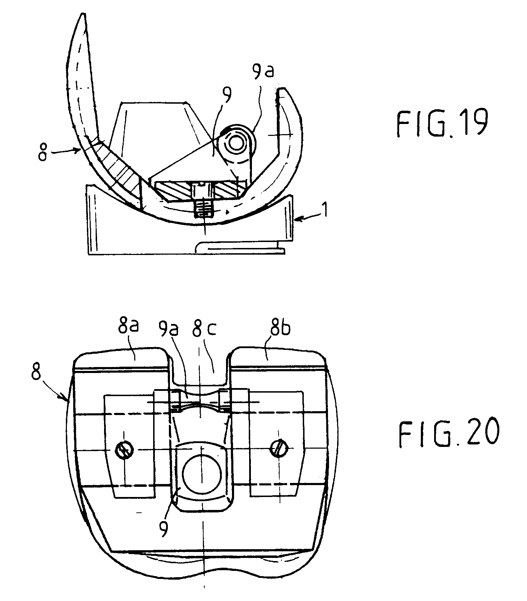

Dans le cas d'une prothèse postéro-stabilisée, l'implant fémoral reçoit au niveau de ses patins condyliens (8a) (8b), un module de stabilisation (9) fixé dans l'épaisseur desdits patins, du côté de leur face interne. Ce module de stabilisation (9) (figures 19 et 20) a une section transversale très sensiblement en forme de U renversé, et présente des agendements (9a), sous forme par exemple d'un rouleau apte à coopérer en appui avec le bossage (1d) du plateau, dans le cas d'une postéro-stabilisée.

Bien évidemment, l'ensemble prothétique, tel que décrit, peut être exécuté dans différentes tailles et présenter tout revêtement de surface approprié.

Les avantages ressortent bien de la description, en particulier on souligne et on rappelle :
- la symétrie de l'embase tibiale, avec l'accouplement non réversible du plateau,
- la possibilité d'équiper la même embase de plusieurs types de quilles,
- la possibilité de fixer la même embase soit avec des chevilles expansives, soit avec des vis d'ostéosynthèse,
- le montage en tiroir du plateau par rapport l'embase, ledit plateau étant parfaitement guidé jusqu'à une position de butée, la fixation étant obtenue par une vis de blocage coopérant avec la quille, pour une sécurité totale,
- la grande surface d'appui du plateau, notamment en partie antérieure, assurant un maximum de stabilité,
- la facilité de pose et dépose éventuelle du plateau par rapport à l'embase, sans aucun dommage pour ledit plateau,
- aucun témoin de position du plateau est nécessaire, étant donné que le verrouillage est irréversible dans les trois dimensions,
- I'évolution du profil de contact fémoral dont la surface d'appui augmente avec la charge appliquée,
- le plateau avec son embase, peut recevoir un implant fémoral de même taille ou de taille immédiatement inférieure ou supérieure.

## Revendications

1. Ensemble prothétique de l'articulation du genou, comprenant un implant fémoral et un implant tibial sous forme d'un plateau en polyéthylène (1), monté en combinaison avec une embase métallique (2), l'ensemble du plateau et de l'embase étant percés coaxialement pour l'engagement d'une vis de blocage (3), caractérisé en ce que l'embase métallique (2) présente un moyen d'accouplement et de guidage (2a) constitué par une nervure (2a) de section transversale profilée en T, formée à partir du bord antérieur de l'embase (2), sur une longueur limitée et apte à coopérer avec une rainure (1a) de forme complémentaire, établie dans l'axe médian de la face de dessous du plateau (1), permettant ainsi l'engagement dudit plateau par simple coulissement, à partir du bord antérieur de l'embase jusqu'à une position de butée.

2. Ensemble selon la revendication 1, caractérisé en ce que la face de dessous de l'embase (2) présente en débordement et en alignement coaxial avec les trous pour le passage de la vis de blocage (3), une portée de centrage creuse (2d) apte à recevoir une quille de stabilité quelconque (4) conformée pour le vissage de ladite vis (3).

3. Ensemble selon la revendication 1, caractérisé en ce que la position dé butée est obtenue par un rebord équerré (2b) formé à partir du bord postérieur de l'embase (2) et coopérant avec une échancrure complémentaire (1b), formée au niveau de la face de dessous du plateau (1).

4. Ensemble selon la revendication 1, caractérisé en ce que la portée de centrage (2d) pour le montage de la quille de stabilité (4), a une forme tronconique, en étant apte à être engagée dans un chambrage (4a) que présente ladite quille, le fond du chambrage étant taraudé pour recevoir la vis de blocage (3).

5. Ensemble selon la revendication 1, caractérisé en ce que dans le cas d'une quille à ailettes (4), l'embase et la quille présentent des moyens complémentaires d'indexation angulaire (4c) (2e).

6. Ensemble selon la revendication 1, caractérisé en ce que la vis de blocage présente une tête (3a) délimitant une portée tronconique, conformée pour être engagée dans un alésage complémentaire de l'embase (2), afin d'être mise en force dans le plateau (1) et participer à son expansion.

7. Ensemble selon la revendication 1, caractérisé en ce que l'embase tibiale (2) est entièrement symétrique et présente des trous (2f) pour l'engagement de chevilles expansives ou de vis d'ostéosynthèse (5).

8. Ensemble selon la revendication 7, caractérisé en ce que les trous reçoivent des rondelles de formes tronconiques (7), pour être emmanchées dans lesdits trous, lesdites rondelles (7) formant chambrage interne pour le logement des têtes de vis d'ostéosynthèse (5).

9. Ensemble selon la revendication 1, caractérisé en ce que l'implant fémoral (8) présente des patins condyliens (8a) (8b) présentant une portée évolutive, d'épaisseur distale et postérieure identique, lesdits patins étant raccordés par une partie (8c) faisant office de trochlée, et qui délimite une surface d'appui concave, convenablement orientée pour recevoir une rotule, afin d'encaisser un maximum d'effort aux moyennes flexions.

10. Ensemble selon la revendication 1, caractérisé en ce que l'implant fémoral (8) reçoit, au niveau de ses patins condyliens (8a) (8b), en débordement de sa face interne, un module de stabilisation (9) apte à coopérer avec une prohéminence médiane, que présente le plateau tibial (1) dans le cas d'une prothèse postéro-stabilisée.

## Claims

1. Prosthesis assembly for the knee joint comprising a femoral implant and a tibial implant in the form of a polyethylene plate (1) mounted in combination with a metal base (2), the plate and base assembly being drilled with a coaxial hole in order to fit a locking screw (3), characterised in that the metal base (2) has a means of connecting and guiding (2a) consisting of a rib (2a) having a T-shaped cross section formed from the anterior edge of base (2) inwards over a limited length and which is adapted to cooperate with a groove (1a) of matching shape along the centre line of the lower surface of plate (1), thus making it possible to engage said plate by simply sliding it in from the anterior edge of the base as far as the end stop.

2. Assembly as claimed in claim 1, characterised in that the lower surface of base (2) has a protruding hollow centring bush (2d) which is coaxially aligned with the holes through which locking screw (3) passes and is adapted to accommodate any stabiliser (4) designed so that said screw (3) can be screwed into it.

3. Assembly as claimed in claim 1, characterised in that the end stop position is obtained by means of a squared edge (2b) formed on the posterior edge of base (2) which cooperates with a matching cut-out (1b) made on the lower surface of plate (1).

4. Assembly as claimed in claim 1, characterised in that the centring bush (2d) used to fit stabiliser (4) has a tapered shape devised to fit into an opening (4a) in said stabiliser, the bottom of the opening being threaded in order to accommodate locking screw (3).

5. Assembly as claimed in claim 1, characterised in that, in the case of a stabiliser with fins (4), the base and the stabiliser have matching means of angular indexing (4c) (2e).

6. Assembly as claimed in claim 1, characterised in that the locking screw has a head (3a) forming a tapered shape devised to fit into a matching bore in base (2) so that it can be force fitted into plate (1) and help expand it.

7. Assembly as claimed in claim 1, characterised in that tibial base (2) is absolutely symmetrical and has holes (2f) into which expanding plugs or osteosynthesis screws (5) can be fitted.

8. Assembly as claimed in claim 7, characterised in that the holes accommodate tapered washers (7) that can be pressed into said holes, said washers (7) forming an internal cavity to accommodate the osteosynthesis screw heads (5).

9. Assembly as claimed in claim 1, characterised in that the femoral implant (8) has condylar pads (8a) (8b) having a varying bearing surface, the distal and posterior thickness being identical, said pads being linked by a part (8c) that acts as a trochlea and defines a concave support surface suitably oriented to accommodate a ball joint in order to absorb maximum force under average flexing.

10. Assembly as claimed in claim 1, characterised in that the femoral implant (8) accommodates, at the level of its condylar pads (8a) (8b) and protruding from its internal surface, a stabiliser module (9) adapted to cooperate with a median projection on the tibial plate (1) in the case of a posterior-stabilised prosthesis.

## Patentansprüche

1. Kniegelenksprothese mit einem femoralen und einem tibialen Implantat in Form eines zusammen mit einer metallischen Grundplatte (2) angebrachten Tibiaplateaus aus Polyethylen (1), wobei Plateau und Grundplatte zwecks Einführen einer Klemmschraube (3) koaxial durchbohrt sind, dadurch gekennzeichnet, daß die metallische Grundplatte (2) eine aus einer Rippe (2a) mit T-förmig profiliertem Querschnitt bestehende Verbindungs- und Führungseinrichtung (2a) aufweist, die ausgehend vom Vorderrand der Grundplatte (2) über eine begrenzte Länge ausgebildet und geeignet ist, mit einer komplementär geformten Rille (1a) in der Mittelachse auf der Unterseite des Plateaus (1) zusammenzuwirken, wodurch das gleitende Aufsetzen des Plateaus vom Vorderrand der Grundplatte her bis zu einer Anschlagstellung ermöglicht wird.

2. Prothese nach Anspruch 1, dadurch gekennzeichnet, daß die Unterseite der Grundplatte (2) in koaxialer Linie mit den Bohrungen für die Durchführung der Klemmschraube (3) einen vorstehenden hohlen Zentrierzapfen (2d) aufweist, der geeignet ist, einen beliebigen Verankerungskiel (4) aufzunehmen, der für das Einschrauben der besagten Schraube (3) ausgebildet ist.

3. Prothese nach Anspruch 1, dadurch gekennzeichnet, daß die Anschlagposition durch einen rechtwinklig aufragenden Vorsprung (2b) erzielt wird, der aus dem hinteren Rand der Grundplatte (2) ausgeformt ist und mit einem ergänzenden Ausschnitt (1b) an der Unterseite des Plateaus (1) zusammenwirkt.

4. Prothese nach Anspruch 1, dadurch gekennzeichnet, daß der Zentrierzapfen (2d) für das Aufsetzen des Verankerungskiels (4) eine kegelstumpfartige Form besitzt und zum Einführen in eine Einsenkung (4a) des besagten Kiels geeignet ist, wobei der Boden der Einsenkung ein Innengewinde für die Aufnahme der Klemmschraube (3) aufweist.

5. Prothese nach Anspruch 1, dadurch gekennzeichnet, daß die Grundplatte und der Kiel im Falle eines Flügelkiels (4) ergänzende Einrichtungen für eine Winkeleinstellung (4c) (2e) aufweisen.

6. Prothese nach Anspruch 1, dadurch gekennzeichnet, daß die Klemmschraube einen Kopf (3a) besitzt, der einen kegelstumpfartigen Zapfen begrenzt und so ausgebildet ist, um in eine ergänzende Bohrung der Grundplatte (2) eingeführt zu werden, damit sie beim Einsetzen in das Plateau (1) unter Kraftaufwand an dessen Expansion beteiligt ist.

7. Prothese nach Anspruch 1, dadurch gekennzeichnet, daß die Tibiagrundplatte (2) vollkommen symmetrisch ist und Löcher (2f) für das Einführen von Spreizdübeln oder Osteosyntheseschrauben (5) aufweist.

8. Prothese nach Anspruch 7, dadurch gekennzeichnet, daß kegelstumpfförmige Unterlegscheiben (7) in die Löcher eingepreßt werden, wobei diese Unterlegscheiben (7) eine Einsenkung für die Aufnahme der Köpfe der Osteosyntheseschrauben (5) bilden.

9. Prothese nach Anspruch 1, dadurch gekennzeichnet, daß das Femurimplantat (8) Kondylenschuhe (8a) (8b) mit einer veränderlichen Auflagefläche bei gleicher distaler und posteriorer Dicke aufweist, wobei die Schuhe durch einen als Trochlea dienenden Teil (8c) miteinander verbunden sind, der eine konkave Auflagefläche begrenzt, die für die Aufnahme einer Kniescheibe passend ausgerichtet ist, um bei mittleren Beugebewegungen ein Maximum an Kraft zu absorbieren.

10. Prothese nach Anspruch 1, dadurch gekennzeichnet, daß das Femurimplantat (8) im Bereich der Kondylenschuhe (8a) (8b) ein aus der Innenseite herausragendes Stabilisierungsmodul (9) besitzt, das geeignet ist, im Falle einer postero-stabilisierten Prothese mit einem mittleren Vorsprung des Tibiaplateaus (1) zusammenzuwirken.
